# EUROPÄISCHE PATENTSCHRIFT

(11) **EP 1 853 209 B1**
(45) Veröffentlichungstag und Bekanntmachung des Hinweises auf die Patenterteilung: **13.05.2009**
(21) Anmeldenummer: 06707268.6
(22) Anmeldetag: 24.02.2006
(51) Int. Cl.: A61F 13/02

(54) **FOLIENVERBAND MIT APPLIKATIONSHILFE**
FILM DRESSING COMPRISING AN APPLICATION AID
ASSEMBLAGE DE FILMS COMPRENANT UN DISPOSITIF D'ASSISTANCE D'APPLICATION

(30) Priorität: 03.03.2005 DE 102005009635
(43) Veröffentlichungstag der Anmeldung: 14.11.2007
(73) Patentinhaber: Paul Hartmann Aktiengesellschaft, 89522 Heidenheim (DE)
(72) Erfinder: EFFING, Jochem, 65779 Kelkheim-Fischbach (DE); ECKSTEIN, Axel, 89522 Heidenheim (DE)
(86) Internationale Anmeldenummer: PCT/EP2006/001732
(87) Internationale Veröffentlichungsnummer: WO 2006/092244

(56) Entgegenhaltungen:
- EP-A- 0 401 949
- EP-A- 0 630 628
- DE-A1- 4 314 834
- US-A- 4 372 303

## Beschreibung

Die Erfindung betrifft einen Folienverband mit einem Polymerfilm und einem Applikationssystem zur leichteren Handhabung des Folienverbandes.

Applikationshilfen für Pflaster oder Wundauflagen sind seit geraumer Zeit bekannt. Insbesondere kommen diese Applikationshilfen bei Film- oder Folienverbänden zum Einsatz. Folienverbände sind dünne, meist transparente, semipermeable Filme oder Folien aus Polymermaterialien. Die Semipermeabilität der Folien verhindert das Eindringen von Bakterien oder Nässe und gestattet dabei einen ausreichenden Sauerstoff- und Wasserdampfaustausch zwischen der abzudeckenden Haut und der äußeren Umgebung des Folienverbandes. Diese Film- oder Folienverbände kommen in vielfältiger Weise zum Einsatz zum Beispiel als Inzisionsfolie zur keimfreien Abdeckung von Operationsstellen, als wasserdichte Abdeckung von Exsudat aufsaugenden Wundauflagen sowie zur Fixierung von Kanülen oder Kathetern. Aufgrund der geringen Dicken dieser Filme oder Folien und der damit verbundenen Instabilität, werden diese Folienverbände mit den unterschiedlichsten Applikationshilfen ausgestattet. Die meisten dieser Applikationshilfen bedienen sich einer zusätzlichen Stützschicht, die während oder nach der Applikation des Folienverbandes entfernt werden.

Filmwundauflagen sind seit geraumer Zeit auch in der Patentliteratur bekannt. So beschreibt die EP 81 990 B1 eine adhäsive Wundauflage, die aus einem dünnen Polymerfilm besteht. Dieser Polymerfilm ist auf einer ersten Seite mit einem auf der Haut klebenden Haftklebstoff beschichtet, der seinerseits mit einer Ablöseschicht abgedeckt ist. Auf der anderen, in Gebrauch körperabgewandten Seite weist der Polymerfilm zur leichteren Handhabung zudem eine leicht zu entfernende Stützschicht aus einem Fasermaterial zum Beispiel ein undurchsichtiges Nonwoven auf. Diese Stützschicht weist dieselbe Größe wie der Polymerfilm auf.

Mit EP 690 706 B1 wird ein klebender Wundverband beschrieben, der zur leichteren Applikation eines Polymerfilms, der von dem Wundverband umfasst ist, eine Trägerschicht aufweist. Diese Trägerschicht deckt vollflächig den Polymerfilm ab und kann in zwei Schritten von dem Polymerfilm entfernt werden. Hierzu wird im ersten Schritt ein Mittelteil aus der Trägerschicht entfernt, worauf im zweiten Schritt ein Rahmenteil entfernt wird. Nachteilig ist, dass die Trägerschicht dieses Wundverbandes nur sehr schwer vom Benutzer ergriffen werden kann.

Des Weiteren beschreibt die EP 951 263 B1 einen klebenden Folienverband, dessen klebende Seite mit einer mindestens zweiteiligen abziehbaren Schutzschicht abgedeckt ist und dessen nicht klebende zweite Seite vollflächig mit einer einteiligen Stützschicht ausgestattet ist Bei diesem Folienverband ist die Stützschicht mit der Schutzschicht an zwei gegenüberliegenden Seiten scharnierartig verbunden, so dass mit dem Entfernen der Schutzschicht gleichzeitig die Stützschicht entfernt werden kann.

Mit der EP 473 918 B1 wird ein Folienverband beschrieben, der eine einteilige Stützfolie aufweist, die ihrerseits an zwei gegenüberliegenden Seiten jeweils eine Griffleiste aufweist. Durch diese Anordnung der Griffleisten, ergibt sich der Nachteil, dass keine Abzugsrichtung für die Stützfolie vorgegeben ist.

Die EP 985 931 A1 beschreibt ein Verband material auf Folienbasis, das im Randbereich der Folie einen nicht klebenden Anfasser aufweist. Die nicht klebende Seite der Folie ist mit einer einteiligen Stützschicht versehen, die die gleiche Größe wie die Folie aufweist und mindestens eine Griffleiste aufweist. Durch Ziehen an dem Anfasser in Verklebungsrichtung kann die applizierte Folie wieder schmerzfrei entfernt werden.

Durch die europäische Patentschrift EP 630 628 B1 ist ein Filmverband bekannt, der zur leichteren Applikation eine zweigeteilte Stützfolie umfasst. Diese Stützfolie ist größer als der zu applizierende Film und bedeckt diesen vollflächig. Zum Entfernen der Stützfolie ist auf der Stützfolie ein weiterer adhäsiv ausgerüsteter Abziehstreifen angebracht, der über der Schnittlinie der Stützfolie angeordnet ist und zum Ergreifen zwei nicht klebende Randbereiche aufweist, die als Griffleiste dienen. Mit Hilfe dieses weiteren Abziehstreifens kann lediglich ein Teil der Stützfolie entfernt werden, worauf der zweite Teil der Stützfolie auf dem Polymerfilm verbleibt.

Mit der WO 97/25012 A1 wird ein Folienverband vorgeschlagen, der vollflächig oder lediglich an zwei gegenüberliegenden Randbereichen der Folie mit einer zweiteiligen Stützschicht versehen ist. Falls die Stützschicht vollflächig auf den Folienverband aufgebracht wird, so können auf der Stützfolie Anfasshilfen aufgebracht sein. Die der Stützschicht gegenüberliegende adhäsive Schutzschicht ist in drei Teile unterteilt.

Darüber hinaus wird mit der europäischen Anmeldung EP 401949 A2 ein klebender Dünnfilm mit einem Applikationssystem für den Film beschrieben. Das Applikationssystem besteht aus zwei Stützfolien, die mittels eines Klebemittels auf der körperabgewandten Seite des Films aufgebracht sind und die an zwei Endbereichen zum leichteren Ergreifen und Entfernen von dem Film frei von dem Klebemittel ausgebildet sind. Hierbei überfängt ein erster Bereich einer ersten Stützfolie einen ersten Bereich einer zweiten Stützfolie.

Mit der US-amerikanischen Patentschrift US 4372303 A wird ein System zur Applikation von Bandagen, Abdeckungen und Wundauflagen beschrieben. Das System besteht aus einem dünnen auf dem Körper eines Patienten zu applizierenden Film, der auf der dem Patienten abgewandten Seite zur leichteren Applikation mit einem Rahmen abgedeckt ist.

Mit der US-amerikanischen Anmeldung US 2004/0162512 A1 ist ein Wundverband bekannt, der eine Trägerschicht aus einem dünnen Polymerfilm und ein radiales Applikationssystem zur leichteren Applikation des Wundverbandes umfasst. Das Applikationssystem ist derart gestaltet, das der Polymerfilm alternierend durch das Applikationssystem abgedeckte und nicht abgedeckte Bereiche aufweist.

Mit der französischen Patentanmeldung FR 2711056 A wird ein absorbierender Wundverband beschrieben, der aus einem dünnen Film, einem Wundkissen und einer Applikationsfolie zur leichteren Applikation des Wundverbandes besteht. Der Wundverband umfasst weiterhin Perforationslinien, mittels derer definierte Bereiche des Films oder Folie von zu applizierenden Bereichen des Films getrennt werden können.

Mit der internationalen Patentanmeldung WO 97/07760 A1 wird ein Wundverband mit Applikationssystem beschrieben. Der Wundverband besteht aus einem klebenden dünnen Film mit einem der klebenden Seite abgewandten Applikationsfolie. Die Applikationsfolie weist an gegenüber liegenden Enden der Folie jeweils zwei Handhabungshilfen zur Ergreifung der Folie auf, wobei die Applikationsfolie kleiner gestaltet ist als der Film.

Diese Schutzrechte zeigen insgesamt verschiedene alternative Lösungen zu Film- oder Folienverbänden mit verschiedenen Applikationssystemen auf. Teilweise werden die mit diesen Schutzrechten als Lösung vorgeschlagenen Folienverbände als zu kompliziert im Aufbau und zu kompliziert in der Anwendung angesehen. Darüber hinaus weisen die mit diesen Schutzrechten vorgeschlagenen Folienverbände mit Applikationshilfen insgesamt eine Steifigkeit auf, die entgegen dem eigentlich zu applizierenden sehr flexiblen Polymerfilm als zu hoch empfunden wird. Diese Flexibilität der Folienverbände ist jedoch erforderlich, um die eigentlich zu applizierenden Polymerfilme zielgenau und faltenfrei zu applizieren.

Aufgabe der vorliegenden Erfindung ist es somit, einen Folienverband mit einem Applikationssystem zur Verfügung zu stellen, der einen einfachen Aufbau aufweist und dennoch eine faltenfreie Applikation des Polymerfilms gewährleistet. Eine weitere Aufgabe der verlegenden Erfindung ist, einen Folienverband bereitzustellen, essen Applikationssystem mindestens zwei Stützfolien umfasst, die nur in einer, unverwechselbaren und unabänderbaren Reihenfolge entfernt werden können. Darüber hinaus besteht eine Aufgabe darin, einen Folienverband zur Verfügung zu stellen, der handhabungssicher und gezielt gemäß seiner vorgesehenen Applikationsfläche angebracht werden kann. Dabei soll der Folienverband ohne jede Form- oder Größeneinschränkung universell einsetzbar sein.

Gelöst wird diese Aufgabe durch einen Folienverband gemäß Anspruch 1. Demnach umfasst der Folienverband einen Polymerfilm und ein Applikationssystem zur leichteren Handhabung des Folienverbandes, wobei das Applikationssystem auf der ersten Seite des Polymerfilms angeordnet ist und mindestens eine erste und eine zweite Stützfolie umfasst, wobei an die erste Stützfolie mindestens eine erste Griffleiste und an die zweite Stützfolie mindestens eine zweite Griffleiste angeformt ist und wobei zumindest die erste Griffleiste eine Grifffläche zum Ergreifen der Griffleiste aufweist und außerdem die erste Griffleiste die zweite Griffleiste zumindest abschnittsweise überfängt.

Jede Griffleiste ist eine zusätzliche Materialkomponente und ist gleichzeitig mit mindestens einer Stützfolie von dem Polymerfilm entfernbar. Dabei ist die durch die Stützfolien abgedeckte Fläche des Polymerfilmes kleiner als die Fläche der ersten Seite des Polymerfilms, wobei die Auflagefläche der Stützfolien insgesamt weniger als 94 % der Fläche der ersten Seite des Polymerfilmes entspricht.

Als Applikationssystem soll bei einer erfindungsgemäßen Ausführung alles verstanden sein, das der leichteren Applikation des Polymerfilms dienen kann und mindestens zwei Stützfolien und zusätzlich zu diesen Stützfolien zumindest zwei Griffleisten umfasst, die an diesen Stützfolien angeformt sind. Unter Anformung soll hier das Verbinden zweier gleicher oder zweier verschiedener Materialien verstanden sein, die mit Hilfe von Klebemitteln, Druck, thermischer Energie, Ultraschallverfahren oder sonstiger Verfahren miteinander lösbar oder unlösbar verbunden sind. Die Griffleiste ist also vorliegend immer eine zusätzliche Materialkomponente, wobei die Griffleiste immer gleichzeitig mit mindestens einer Stützfolie von einem Polymerfilm entfernbar ist. Des Weiteren soll des leichteren Verständnisses wegen im Zusammenhang mit der vorliegenden Erfindung unter einem Film oder Polymerfilm immer der eigentlich zu applizierende Film oder Polymerfilm wie zum Beispiel ein Wundverband verstanden sein; dagegen soll unter einer Folie oder Polymer- bzw. Stützfolie immer ein Teil des Applikationssystems verstanden sein, das heißt die Unterscheidung zwischen Film und Folie ist hier lediglich auf die Funktion der Bestandteile zu verstehen. Hinsichtlich des Materials soll, wie im Sprachgebrauch üblich, kein Unterschied zwischen einem Film und einer Folie verstanden sein.

Durch diese Anordnung der Griffleisten ist in besonders einfacher Weise gewährleistet, dass der Anwender in jedem Fall nur die zu oberst liegende erste Griffleiste als erste Griffleiste betätigen kann und somit eine erste Stützfolie als erste Folie von dem Polymerfilms entfernen kann. Erst im zweiten Schritt kann der Anwender daraufhin eine zweite Stützfolie mit Hilfe der zweiten Griffleiste entfernen. Es ist somit eine Reihenfolge bei der Entfernung der Stützfolien vorgegeben und ein besonders handhabungssicherer Folienverband bereitgestellt.

In einer ersten bevorzugten Ausführungsform eines erfindungsgemäßen Folienverbandes ist vorgesehen, dass die erste Griffleiste eine zum Ergreifen durch den Benutzer bestimmte Grifffläche, vorzugsweise ausgeführt als Hintergriffsmittel, von mindestens 2 cm², insbesondere mindestens 5 cm² und ganz besonders bevorzugt von mindestens 7 cm² aufweist. Insbesondere ist vorgesehen, dass die erste Griffleiste die zweite Griffleiste vollständig überfängt. Als besonders handhabungssicher hat sich herausgestellt, wenn die erste Griffleiste eine freie Grifffläche von mindestens 2 cm², insbesondere 4 cm² und ganz besonders bevorzugt von 6 cm² aufweist. Diese freie Grifffläche ist vorliegend der Teil der Grifffläche, die über die zweite Griffleiste seitlich übersteht.

In einer weiter bevorzugten Ausführungsform eines erfindungsgemäßen Folienverbandes ist vorgesehen, dass die zweite Griffleiste eine zum Ergreifen durch den Benutzer bestimmte Grifffläche, vorzugsweise ausgeführt als Hintergriffsmittel von mindestens 2 cm², insbesondere mindestens 3 cm² und ganz besonders bevorzugt von mindestens 4 cm² aufweist.

In einer weiteren Ausführung der Erfindung ist vorgesehen, dass die durch die Stützfolien abgedeckte Fläche des Polymerfilms kleiner ist als die Fläche der ersten Seite des Polymerfilms. Somit weist der Polymerfilm vorzugsweise mindestens einen stützfolienfreien Bereich auf. Insbesondere weist der Folienverband in dieser Ausgestaltung zwei oder mehr Stützfolien auf, deren Auflagefläche insgesamt weniger als 97 % und insbesondere weniger als 94 % der Fläche der ersten Seite des zu applizierenden Polymerfilms entspricht.

Ein Vorteil eines solchen Folienverbands mit mindestens einem stützfolienfreien Bereich ist, dass dieser Bereich des Polymerfilms, der nicht durch eine Stützfolie abgedeckt wird, aufgrund der leichteren Biegsamkeit im Vergleich zum Polymerfilm mit Stützfolie, während der Applikation des Folienverbandes als Gelenk fungieren kann. Somit kann als Stützfolie auch ein relativ steifes Material zum Einsatz gebracht werden, wobei gleichzeitig eine passgenaue Applikation gewährleistet ist. Bei einer bekannten mehrteiligen Stützfolie, die insgesamt den Polymerfilm vollflächig abdeckt, muss die Wahl des Stützmaterials auf relativ flexible Materialien eingeschränkt werden, um eine passgenaue Applikation des Polymerfilms zu gewährleisten.

Insbesondere kann ein erster nicht durch die Stützfolien abgedeckter Bereich an einem Rand des Polymerfilms angeordnet sein. Als Rand soll hier jeder Bereich einer Fläche verstanden sein, der sich von der Kante einer Fläche in das Innere einer Fläche erstreckt, wobei die flächenhafte Ausdehnung des Randes kleiner ist als 50% der gesamten Fläche. Hierdurch wird ein Folienverband bereitgestellt, der vorteilhafter Weise einen Bereich aufweist, der eine durch den Polymerfilm selbst vorgegebene Flexibilität aufweist und eine leichte Erstfixierung des zu applizierenden Films gewährleistet, wobei gleichzeitig durch die Stützfolien auf den weiteren Bereichen eine sichere Handhabung gewährleistet ist. Hierbei hat sich als besonders gut handhabbar und anwendungssicher herausgestellt, wenn eine der Stützfolien in mindestens einem Punkt ihrer äußeren Kante einen Abstand von der äußeren Kante des Polymerfilms von mindestens 2 mm, insbesondere mindestens 3 mm und ganz besonders mindestens 5 mm besitzt. Besonders bevorzugt ist ein Abstand, der in jedem Punkt der Kante der Stützfolie einen gleichen Abstand von mindestens 2 mm, insbesondere mindestens 3 mm und ganz besonders mindestens 5 mm zu der äußeren Kante des Polymerfilms aufweist.

In einer weiteren Ausgestaltung der Erfindung ist vorgesehen, dass eine Griffleiste einen nicht durch die Stützfolien abgedeckten Bereich des Polymerfilms zumindest abschnittsweise überfängt. Es kann auch vorgesehen sein, dass die Griffleiste den nicht durch die Stützfolie abgedeckten Bereich vollständig überfängt. Weiterhin kann das Applikationssystem insgesamt dieselbe Größe wie der Polymerfilm aufweisen. Hierbei ist mit derselben Größe, eine Größe hinsichtlich der Auflagefläche gemeint, das heißt die Umfangsbegrenzungen des Applikationssystems und des Polymerfilmes sind bündig. Durch dieselbe Größe des Applikationssystems und des Polymerfilms und dadurch, dass die Griffleiste lediglich an der Stützfolie angeformt ist und keine Verbindung zu dem Polymerfilm aufweist, ist gewährleistet, dass neben der bereits geschilderten hohen Flexibilität in dem nicht durch die Stützfolie abgedeckten Bereich zudem der gesamte Film abgedeckt ist und somit auch während der Applikation vollständig geschützt ist.

In Weiterbildung des Erfindungsgedankens kann der Folienverband einen ersten nicht durch die Stützfolien abgedeckten Bereich zwischen den Stützfolien aufweisen. Der Abstand der beiden Stützfolien zueinander beträgt dabei vorzugsweise in jedem Punkt mindestens 2 mm, insbesondere 3 mm und ganz besonders 5 mm. Besonders bevorzugt ist ein Applikationssystem, das zwei Stützfolien aufweist, die in jedem Punkt den gleichen Abstand zueinander aufweisen. Als besonders handhabungssicher hat sich auch herausgestellt, wenn die erste Griffleiste sowohl den nicht abgedeckten Bereich des Polymerfilms als auch die zweite Griffleiste vollständig überfängt. Hierdurch ist gewährleistet, dass der Anwender in jedem Fall nur die zu oberst liegende erste Griffleiste als erste Griffleiste betätigen kann und somit die erste Stützfolie als erste Folie von dem Polymerfilm entfernen kann und der gesamte Polymerfilm durch das Applikationssystem abgedeckt ist.

Falls der Folienverband ein Applikationssystem mit zwei Stützfolien aufweist und ein erster stützfolienfreier Bereich zwischen den Stützfolien vorgesehen ist, kann getrennt von diesem ersten nicht abgedeckten Bereich ein zweiter Bereich vorgesehen sein, der ebenfalls nicht durch eine Stützfolie überdeckt wird. Dieser zweite Bereich kann weiterhin vorzugsweise durch eine Griffleiste überdeckt sein. Es kann aber auch vorgesehen sein, dass dieser zweite Bereich weder von einer Stützfolie noch von einer Griffleiste überdeckt wird. Vorzugsweise ist dieser zweite nicht abgedeckte Bereich des Polymerfilms an einem Rand des Folienverbandes angeordnet. Auf diese Weise weist der Folienverband sowohl ein Gelenk innerhalb des Verbandes auf als auch einen Bereich zur Erstfixierung auf.

Falls ein Applikationssystem vorgesehen ist, das mehr als zwei Stützfolien umfasst, kann jeder Stützfolie eine Griffleiste zugeordnet sein. In einer besonders bevorzugten Ausführung können aber auch einer Griffleiste zwei Stützfolien zugeordnet sein. Insbesondere können in einem Folienverband mit drei Stützfolien einer Griffleiste zwei Stützfolien zugeordnet sein. Durch diese Anordnung bzw. Zuordnung der Griffleisten auf den Stützfolien können zwei separate Stützfolien in einem Arbeitsschritt entfernt werden. Als Stützfolien sind besonders transparente oder transluzente Folienmaterialien vorgesehen. Alternativ können jedoch auch opake oder undurchsichtige Folienmaterialien verwendet werden. Insbesondere kommen als Stützfolie solche Folien zur Verwendung, die aus Polyester, Polyethylen, Polypropylen, Polyvinylchlorid, Polystyrol, Polyamid, Polykarbonat, Celluloseester, Ethylenvinylacetat, Polyvinylacetat, Polyvinylalkohol und/oder Mischungen hiervon gefertigt werden. Besonders bevorzugt sind Stützfolien aus transparentem Polyester oder Polyethylen oder Polypropylen. Dabei hat es sich weiterhin als besonders vorteilhaft erwiesen, wenn die Dicken der Stützfolie so eingestellt werden, dass diese eine Dicke von 15 bis 80 µm, insbesondere von 20 bis 60 µm und ganz bevorzugt von 20 bis 40 µm aufweisen.

Zur Herstellung einer Griffleiste können dieselben Materialien verwendet werden, die als Stützfolie zur Anwendung gebracht werden. In einer besonders bevorzugten Ausführungsform wird die Griffleiste aber aus einem Folienmaterial hergestellt, das flexibler ist als die Stützfolie. Falls ein Applikationssystem vorgesehen ist, das zwei oder mehr Stützfolien und zwei oder mehr Griffleisten umfasst, sind alle Griffleisten aus einem Material gefertigt, das flexibler ist als jede Stützfolie. Hierdurch ist gewährleistet, dass die Griffleisten besonders leicht zu ergreifen sind. In einer weiteren besonders bevorzugten Ausführungsform mit zwei Griffleisten ist vorgesehen, dass die Griffleiste der ersten Stützfolie flexibler ist als die Griffleiste der zweiten Stützfolie. Dabei ist es weiterhin vorteilhaft, wenn die erste Griffleiste vollständig von der zweiten Griffleiste übergriffen wird.

Zusätzlich kann bei einem System mit zwei Griffleisten ein Aktivierungsmittel vorgesehen sein, dass zwischen der ersten und der zweiten Griffleiste angeordnet ist. Dieses Aktivierungsmittel kann zum Beispiel ein zusätzlicher adhäsiver Streifen sein, der zu beiden Seiten seiner Auflageflächen eine unterschiedliche Klebekraft aufweist. Bei der Anwendung eines solchen Folienverbandes kann dann zum Beispiel eine über der zweiten Griffleiste angeordnete erste Griffleiste ergriffen werden und mit dieser Griffleiste das Aktivierungsmittel und eine Stützfolie von dem Polymerfilm entfernt werden, wobei gleichzeitig die zweite Griffleiste so aktiviert wird bzw. aufgerichtet wird, dass diese von dem Anwender leichter greibar ist.

Als Polymerfilms können in einem erfindungsgemäßen Folienverband insbesondere Polymerfilme eingesetzt werden, die eine hohe Wasserdampfdurchlässigkeit aufweisen. Hierzu sind besonders Filme geeignet, die aus Polyurethan, Polyetherurethan, Polyesterurethan, Polyether-Polyamid-Copolymeren, Polyacrylat oder Polymethacrylat gefertigt werden. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 µm, insbesondere 20 bis 40 µm und ganz besonders bevorzugt von 25 bis 30 µm aufweisen. Die Wasserdampfdurchlässigkeit des Polymerfilms in einem erfindungsgemäßen Folienverbande weist vorzugsweise mindestens 750 g/ m²/ 24 Std., insbesondere mindestens 1000 g/ m²/ 24 Std. und ganz besonders bevorzugt mindestens 2000 g/ m²/ 24 Std. auf (gemessen nach DIN 13726).

Auf der dem Applikationssystem gegenüberliegenden zweiten Seite des zu applizierenden Polymerfilms kann ein Klebemittel aufgebracht sein. Dieser Auftrag kann sowohl vollflächig als auch diskontinuierlich oder nur in bestimmten Bereichen erfolgen. Bei dem verwendeten Klebemittel kann es sich um einen üblichen Haftkleber, insbesondere um einen Acrylathaftkleber oder einen druckempfindlichen Haftkleber auf Basis von Polyurethanen handeln. Bevorzugt handelt es sich um Gelhaftkleber, insbesondere auf Basis von Polyurethanen, insbesondere wässrigen Polyurethanen. Ganz besonders bevorzugt handelt es sich um Hydrogelhaftkleber, insbesondere auf Basis von wässrigen Acrylaten.

Vorteilhafterweise beträgt das Flächengewicht des Klebemittels 20-100 g/m², insbesondere 35-50 g/m², wobei das Klebemittel diskontinuierlich, vorzugsweise aber vollflächig aufgetragen sein kann.

Die Wasserdampfdurchlässigkeit des mit dem Klebemittel versehenen Polymerfilm beträgt vorzugsweise mindestens 1000 g/ m²/ 24 Std., besonders bevorzugt mindestens 1200 g/ m²/ 24 Std. und ganz besonders bevorzugt mindestens 2000 g/ m²/ 24 Std. (gemessen nach DIN EN 13726) aufweisen.

Gemäß einer Weiterführung der Erfindung kann der Folienverband auf der dem Applikationssystem gegenüberliegenden zweiten Seite des Polymerfilms vollflächig mit einem Haftklebemittel beschichtet sein und das Haftklebemittel mit einer Abdeckfolie oder einem Abdeckpapier geschützt sein. Als Abdeckschicht kann dabei jedes handelsübliche Silikonpapier oder -folie sowie eine mit einer Fluorverbindung beaufschlagtes Papier oder Folie verwendet werden.

Falls der Folienverband als Wundauflage gefertigt werden soll, ist gemäß einer weiteren Ausführungsform vorgesehen ein Wundpad oder Wundkissen auf der zweiten , in Gebrauch körperzugewandten Seite des Polymerfilms anzuordnen. Ein solcher Folienverband ist besonders als Wundauflage geeignet, wenn das Wundpad oder -kissen adhäsiv mit dem Polymerfilm verbunden ist. Dieses Wundkissen kann aus einem Vlies also einem Nonwoven gefertigt sein. Vorzugsweise kann es sich bei dem Vlies um ein hydrophiles Fasermaterial wie zum Beispiel Baumwolle, Viskose, Cellulose und Polyester oder deren Mischungen mit vorzugsweise hydrophilisiertem Polymethylen oder Polypropylen handeln.

Insbesondere kann an Stelle des Wundkissens oder zusätzlich zum Wundkissen der Folienverband auf der zweiten, in Gebrauch körperzugewandten Seite des Polymerfilms eine die Wundheilung fördernde Schicht aufgebracht sein. Unter einer wundheilungsfördernden Schicht soll hierbei jede Schicht verstanden sein, die zur Anwendung in der feuchten Wundbehandlung zu verwenden ist. Insbesondere sind hierzu Hydrogele auf Basis von Polyurethanen, Acrylaten oder wasserlöslichen Cellulosen oder Mischungen hiervon bevorzugt, die einen Wasseranteil von mindestens 50 % bezogen auf das Gesamtgewicht des Hydrogels aufweisen. Diese Hydrogele können sowohl auf das Wundkissen als auch auf die zweite Seite des Polymerfilmes direkt aufgebracht sein.

Um einen anwendungssicheren Folienverband bereit zu stellen, muss eine genaue Abstimmung der verwendeten Materialien erfolgen. Insbesondere müssen die verwendeten Materialien hinsichtlich ihrer Release-Eigenschaften abgestimmt werden. Diese durch zusätzliche Mittel einstellbaren Release-Eigenschaften beruhen auf den Kräften, die zwischen zwei verwendeten Materialien herrschen. So kann zum Beispiel durch eine gezielte Oberflächenbehandlung eines Materials eine anziehende oder eine abstoßende Wirkung auf ein zweites mit diesem ersten Material zusammen zu bringendes Material eingestellt werden. Eine Oberflächenbehandlung, die eine anziehende Wirkung zwischen zwei Materialien hervorruft, kann zum Beispiel durch einen zusätzlichen adhäsiven Auftrag, eine statische Aufladung oder durch ein Verschmelzen der beiden zusammenzubringenden Materialien erfolgen. Eine abstoßende Wirkung kann zum Beispiel durch eine zusätzliche Schicht auf einem Material aus Silikon- oder Fluorverbindungen hervorgerufen werden. Als Release-Kraft (Abziehkraft) wird dabei eine solche Kraft verstanden, die notwendig ist zwei Materialien voneinander zu trennen (gemessen nach DIN 53530).

In einer weiteren Ausführungsform des erfindungsgemäßen Folienverbandes sind diese Release-Eigenschaften so eingestellt, dass die Abziehkraft, die notwendig ist um eine Abdeckfolie oder -papier von dem zu applizierenden Polymerfilm zu lösen, größer ist als die Abziehkraft, die notwendig ist, um die Stützfolie oder die Stützfolien von dem Polymerfilm zu trennen.

Bei einer Ausgestaltung des Folienverbandes mit zwei Stützfolien sind die Release-Eigenschaften bevorzugt so eingestellt, dass die Abziehkraft, die notwendig ist um die erste Stützfolie von dem zu applizierenden Polymerfilm zu lösen, gleich groß ist wie die Abziehkraft, die zum Lösen der zweiten Stützfolie notwendig ist.

Weiterhin bevorzugt sind bei einem Folienverbund mit zwei Stützfolien und zwei Griffleisten die Release-Eigenschaften so eingestellt, das die Abziehkraft, die notwendig ist, um die erste Griffleiste von der zweiten Griffleiste oder die zweite von der ersten Griffleiste zu lösen, geringer ist als die Ablösekraft, die notwendig ist, um die Stützfolie von dem zu applizierenden Polymerfilm zu lösen.

Bei einer weiteren Ausgestaltung des Folienverbandes mit zwei Stützfolien sind die Release-Eigenschaften bevorzugt so eingestellt, das die Abziehkraft, die notwendig ist, um die erste Stützfolie von dem zu applizierenden Polymerfilm zu lösen, größer ist als die Abziehkraft, die zum Lösen der ersten Griffleiste von der zweiten Griffleiste notwendig ist

Die Haftung der Stützfolie auf dem Polymerfilm ist vorzugsweise nur 0,01 bis 0,5 N/ 25 mm, ganz besonders bevorzugt 0,01 bis 0,1 N/ 25 mm, gemessen nach DIN 53530. Hierzu, wird das Stützmaterial vorzugsweise direkt bei der Herstellung des Polymerfilms auf den Polymerfilm aufgebracht, bzw. der Polymerfilm direkt auf dem Stützmaterial erzeugt Hierbei können alle üblichen Verfahren zur Filmherstellung angewendet werden zum Beispiel durch Giessen, Rakeln, Extrudieren oder andere bekannte Methoden der Film- oder Folienherstellung. Falls notwendig, kann das Stützmaterial auf der Beschichtungsseite aufgeraut oder einer anderen haftungsfördernden Behandlung unterworfen werden. Auch eine adhäsionsfördernde Beschichtung kann vorteilhaft sein.

Wichtig dabei ist, dass die Haftung des Polymerfilms auf der zu applizierenden Oberfläche wesentlich größer ist als die Haftung einer Stützfolie an dem Polymerfilm.

In einer besonderen Ausgestaltung der Erfindung ist vorgesehen, dass ein Folienverband umfassend einen Polymerfilm mit einem Applikationssystem in einer Verpackung angeordnet ist. Insbesondere ist dabei vorgesehen, dass die Verpackung eine sterile Verpackung ist.

Hervorzuheben ist an dieser Stelle, dass die hier aufgeführten Merkmale der alternativen Ausgestaltungen der Erfindungen nicht auf die einzelnen Alternativen zu beschränken sind. Es ist vielmehr der Fall, dass die Kombination der Ausgestaltungen bzw. die Kombination der Einzelmerkmale der alternativen Formen ebenso zu einer erfindungsgemäßen Ausgestaltung zu zählen ist. Ebenso wenig soll die Erfindung durch die nachfolgende Beschreibung der Zeichnungen reduziert verstanden sein.

Nachstehend wird die Erfindung unter Bezugnahme auf die Zeichnung erläutert. In den Zeichnungen zeigt:
- Figur 1:: ein erfindungsgemäßer Folienverbandes, in Aufsicht
- Figur 2:: die in Figur 1 wiedergegebene Ausführungsform eines Folienverbandes, im Querschnitt
- Figur 3:: eine weitere Ausführungsform eines erfindungsgemäßen Folienverbandes, in Aufsicht
- Figur 4:: die in Figur 3 wiedergegebene Ausführungsform eines Folienverbandes, im Querschnitt
- Figur 5 a - c:: ein erfindungsgemäßer Folienverband bei der Verwendung, im Querschnitt

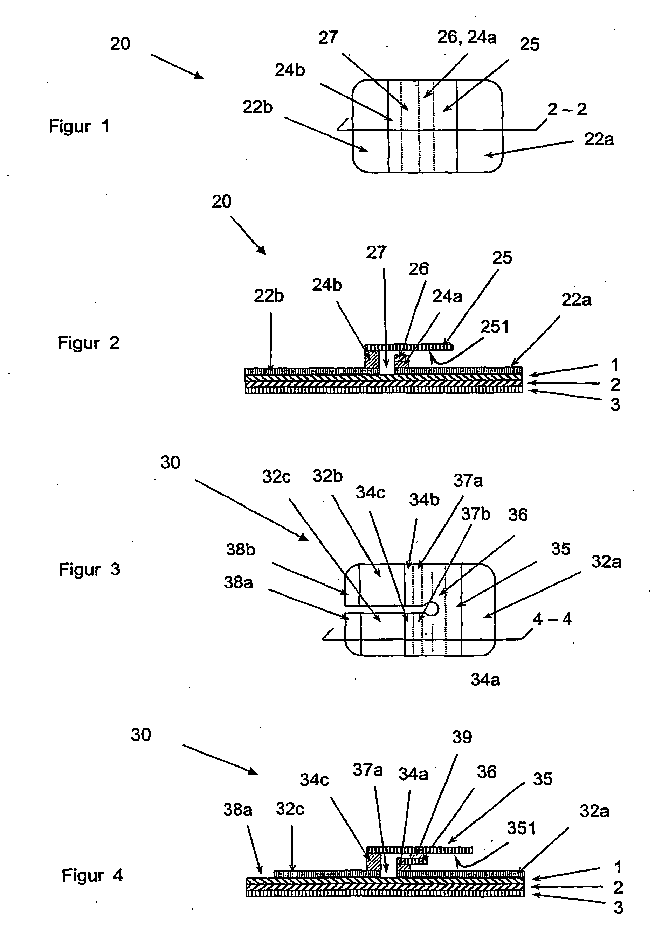

Mit Figur 1 und Figur 2 ist eine erste Ausführungsform der vorliegenden Erfindung gezeigt. Dieser Folienverband (20) ist von rechteckiger Grundform und besteht aus einem Polymerfilms (1), einem auf der zweiten Seite des Polymerfilms aufgebrachten vollflächigen Klebstoffauftrag (2) und einer diesen Klebstoff abdeckenden Abdeckschicht (3). Auf der ersten Seite des Polymerfilms weist dieser ein Applikationssystem auf, das zwei Stützfolien (22a, 22b), zwei Griffleisten (25, 26) sowie zwei Adhäsive (24a, 24b) umfasst. Die beiden Stützfolien sind derart auf den Polymerfilms aufgebracht, dass dieser bis auf den nicht abgedeckten Bereich (27) vollständig von den Stützfolien abgedeckt wird. Der abgedeckte Bereich des Polymerfilmes beträgt etwa 96 % der Fläche der ersten Seite des Polymerfilmes. In dieser Ausführung überfängt die erste Griffleiste (25), die mittels des ersten Adhäsivs (24b) auf der ersten Stützfolie (22b) aufgeklebt ist, sowohl den stützfolienfreien Bereich (27) als auch die zweite Griffleiste (26), die mittels des zweiten Adhäsivs (24a) auf der zweiten Stützfolie (22a) aufgeklebt ist. Die erste Griffleiste (25) weist zum Ergreifen durch den Benutzer eine als Hintergriffsmittel ausgebildete Grifffläche auf. Der äußere Teil der Grifffläche ragt seitlich als freie Grifffläche (251) über die zweite Griffleiste (26) hinaus. Diese zweite Griffleiste (26) weist in der dargestellten Ausführungsform keine als Hintergriffsmittel ausgebildete Grifffläche auf. Dies ist aber wie in Figuren 3 und 4 bei einem Kanülenpflaster dargestellt ebenfalls denkbar und vorteilhaft, um das Ergreifen der zweiten Griffleiste (26) zu erleichtern. Dadurch, dass nur eine Griffleiste greifbar und für den Benutzer sichtbar ist, ist eine Reihenfolge beim Entfernen der beiden Stützfolien vorgegeben.

Mit Figur 3 und Figur 4 ist ein weiterer Folienverband (30) wiedergegeben. Dieser Folienverband kann als Kanülen- oder Katheterpflaster verwendet werden. Der Folienverband weist eine rechteckige Grundform auf, deren kurze Seite parallel zur langen Seite eine Aussparung aufweist. Durch diese Aussparung erhält der Folienverband zwei voneinander unabhängige Bereiche, die über einen dritten Bereich miteinander verbunden sind und die bei der Fixierung, zum Beispiel einer Kanüle jeweils auf einer Seite der Kanüle auf einer Oberfläche fixiert werden können. Der Folienverband weist einen Polymerfilm (1), eine Acrylat-Klebeschicht (2) und eine die klebende Schicht abdeckende Abdeckschicht (3) auf. Auf der der klebenden Schicht abgewandten ersten Seite des Polymerfilms ist ein Applikationssystem angeordnet. Dieses Applikatiönssystem umfasst zwei Griffleisten (35, 36), die mittels dreier Adhäsive (34a, 34b, 34c) auf drei Stützfolien (32a, 32b, 32c) aufgebracht sind. Die erste Griffleiste (35) ist sowohl der ersten Stützfolie (32c) als auch der zweiten Stützfolie (32b) zugeordnet mithin daran angeformt. Hierdurch können diese beiden Stützfolien (32c, 32b) mit einem Griff entfernt werden. Die erste Griffleiste (35) überfängt sowohl die zentralen Bereiche des Polymerfilmes, die nicht durch Stützfolien abgedeckt sind (37a, 37b) als auch die zweite Griffleiste (36). Der äußere Teil der Grifffläche ragt seitlich als freie Grifffläche (351) über die zweite Griffleiste (26) hinaus, so dass auch hier eine Reihenfolge beim Entfernen derselben vorgegeben ist.

Die Stützfolien bedecken insgesamt eine Fläche des Polymerfilms, die 92% der Fläche der ersten Seite des Polymerfilms beträgt, da neben den zentralen Bereichen (37a, 37b), die nicht durch Stützfolien abgedeckt sind, auch in den beiden Randbereichen (38a, 38b) keine Stützfolie angeordnet ist. Diese stützfolienfreien Randbereiche können nach dem Entfernen der Abdeckschicht zur Erstfixierung verwendet werden. Mit Figur 4 ist zudem ein Aktivierungsmittel (39) zwischen den beiden Griffleisten wiedergegeben. Dieses Aktivierungsmittel (39) ist wie auch das Adhäsiv zur Befestigung der Griffleiste (36) auf der Stützfolie (32a) in Figur 3 nicht dargestellt. Dieses Aktivierungsmittel (39) ist ein doppelseitiges Klebeband, das zur ersten Griffleiste (35) eine höhere Klebkraft aufweist als zur zweiten Griffleiste (36). Damit wird bei der Entfernung der Stützfolien (32b, 32c) mittels der ersten Griffleiste (35) die darunter liegende zweite Griffleiste (36) aufgerichtet, bevor die Klebkraft zwischen der ersten Griffleiste (35) und dem Aktivierungsmittel (39) aufgehoben wird. Die zweite Griffleiste (36) ist somit im nächsten Schritt zur Entfernung der Stützfolie (32a) leichter greifbar.

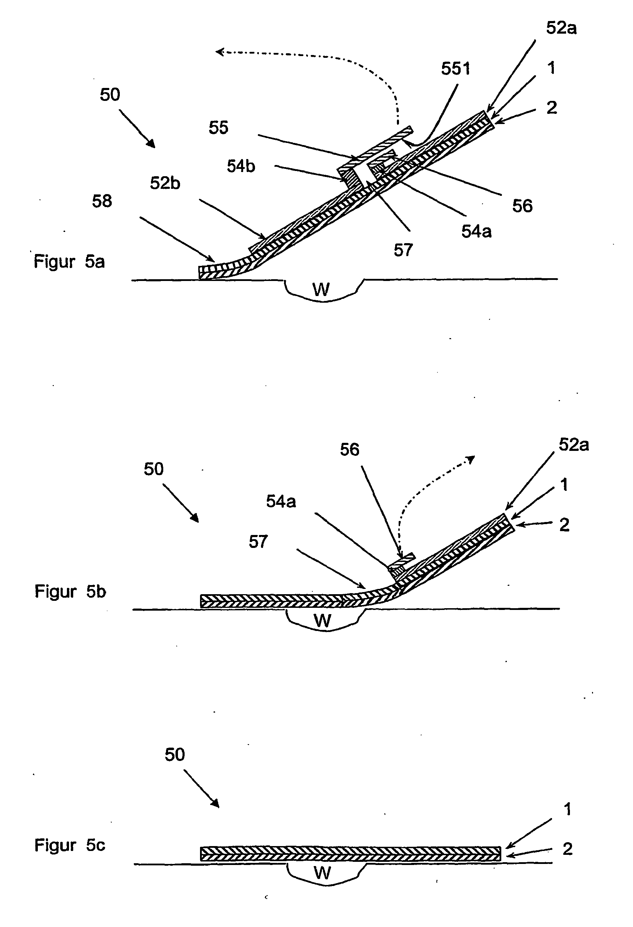

Figuren 5a bis 5c zeigen die Anwendung eines erfindungsgemäßen Folienverbandes mit zwei stützfolienfreien Bereichen nach Entfernen der Abdeckschicht (3) von der Klebstoffschicht (2). Wie in den Figuren 5a bis 5c gezeigt ist, kann daraufhin, um einen Folienverband (30, 50) zum Beispiel oberhalb einer Wunde (W) zu applizieren, ein erster stützfolienfreier Randbereich (38a, 38b, 58) auf einem an die Wunde (W) angrenzenden Bereich fixiert werden. Durch die hohe Flexibilität des Polymerfilms ohne Stützfolie ist dies sehr gut möglich. Im weiteren Schritt kann der Anwender dann durch die vorgegebene Anordnung der Griffleisten (35, 36, 55, 56), die mittels adhäsiver Klebstoffe (34a, 34b, 34c, 54a, 54b) auf den jeweiligen Stützfolien aufgebracht sind, den zu applizierenden Polymerfilm (1) genau über der Wunde platzieren. Durch den weiteren nicht durch Stützfolien abgedeckten Bereich (37a, 37b, 57) weist der Folienverband eine Art Gelenk auf, das eine faltenfreie Applikation gestattet. Die Stützfolien (32a, 32b, 32c, 52a, 52b) können nacheinander während der Applikation oder nacheinander nach erfolgter Applikation des Polymerfilms entfernt werden, wobei zunächst die erste Griffleiste (35, 55) über ihre freie Grifffläche (351, 551) ergriffen und damit zuerst die erste Stützfolie (32b, 32c, 52b) abgezogen werden kann.

### Ausführungsbeispiel 1:

Der Folienverband weist eine rechteckige Grundform mit einer Kantenlänge von 57 x 80 mm auf (Auflagefläche 45,6 cm²). Er umfasst einen transparenten Polyetherurethanfilm, der auf seiner in Gebrauch körperzugewandten Seite mit einem Hydrogel-Klebstoff auf Acrylat-Basis beschichtet ist. Der Klebstoff ist in einem vollflächigen Auftrag in einer Menge von ca. 35 g/ m² auf den ca. 25 µm dicken Polymerfilm (gemessen bei einem Prüfdruck von 0,5 kPa) aufgebracht. Der Polymerfilm weist zusammen mit dem Klebstoff eine Wasserdampfdurchlässigkeit von ca. 2600 g/ m²/ 24 Std. auf (gemessen nach DIN EN 13726, mit dem Unterschied, das nach 4 Std. Messzeit abgebrochen wurde und das ermittelte Ergebnis auf 24 Std. extrapoliert wird). Ein solcher Polymerfilm ist unter dem Handelsnamen Inspire 6200 bei der Fa. InteliCoat Technologies, Wrexham Industrial Estate, Wrexham LL13 9UF, UK, erhältlich. Die klebende Seite dieses Polymerfilms ist mit einem silikonisierten Abdeckpapier erhältlich unter dem Handelsnamen Separacon 980-60 bei der Fa. Maria Soell GmbH & Co. KG, Frankenstrasse 45, D-63667 Nidda-Eichelsdorf, abgedeckt. Auf der anderen, in Gebrauch körperabgewandten Seite des Polymerfilmes ist ein Applikationssystem angeordnet, das aus zwei Stützfolien besteht, auf denen jeweils eine Griffleiste angeordnet ist. Die Stützfolien sind wie in Figur 1 und Figur 2 dargestellt auf dem Polymerfilm angeordnet. In dem hier vorliegenden Folienverband ist jedoch zusätzlich ein Randbereich verwirklicht, der durch keine Stützfolie und keine Griffleiste abgedeckt ist. Dieser zusätzliche stützfolienfreie Randbereich ist auf der kurzen Seite des Rechtecks angeordnet und weist eine gleichförmige Breite von 5 mm auf. Die beiden Stützfolien sind gleich groß und weisen eine Kantenlänge von 57 x 36 mm auf (Auflagefläche: 2 x 20,5 cm² = 41,0 cm²). Der Abstand der beiden Folien beträgt in jedem Punkt ihrer zueinander parallel liegenden gleich langen Kanten ca. 3 mm. Damit ergibt sich insgesamt für beide Stützfolien eine Auflagefläche von 90 % bezogen auf Fläche der ersten Seite des Polymerfilms. Die Stützfolien sind aus einem 30 µm dicken Polyesterfilm gefertigt (gemessen bei einem Prüfdruck von 0,5 kPa). Auf jeder Stützfolie ist mittels eines Acrylat-Klebstoffes eine Griffleiste aufgeklebt. Die Griffleisten weisen insgesamt eine Anordnung auf, wie sie mit Figur 4 wiedergegeben ist, wobei die erste mit Bezugszeichen (35) skizzierte Griffleiste eine Größe von 57 x 39 mm aufweist und über die gesamte Breite (57 mm) mit der zugehörigen Stützfolie verbunden ist. Die zweite mit Bezugszeichen (36) wiedergegeben Griffleiste weist eine Größe von 57 x 22 mm auf. Beide Griffleisten sind jeweils über einen 5 mm breiten adhäsiven Verbindungsstreifen mit der jeweiligen Stützfolie verbunden und sind aus einer 20 µm dicken transparenten Polyesterfolie gefertigt. Somit hat die erste Griffleiste eine Grifffläche, deren gleichförmige Breite 34 mm beträgt. Die Größe der Grifffläche der ersten Griffleiste beträgt 19,4 cm². Die gleichförmige Breite der Grifffläche der zweiten Griffleiste beträgt 17 mm. Somit beträgt die Größe der Grifffläche der zweiten Griffleiste 9,7 cm². Die gleichförmige Breite des über die zweite Griffleiste überstehenden Teils der ersten Grifffläche, also die Breite der freien Grifffläche der ersten Griffleiste bemisst sich mit 9 mm. Die Größe der freien Grifffläche beträgt somit 5,1 cm².

### Ausführungsbeispiel 2:

Der Folienverband weist eine rechteckige Grundform mit einer Kantenlänge von 57 x 80 mm auf (Auflagefläche 45,6 cm²). Er umfasst einen transparenten Polyetherurethanfilm, der auf seiner, in Gebrauch körperzugewandten Seite mit einem druckempfindlichen Haftklebstoff auf Acrylat-Basis beschichtet ist. Der Klebstoff ist in einem vollflächigen Auftrag in einer Menge von ca. 25 g/ m² auf den ca. 30 µm dicken Polymerfilm (gemessen bei einem Prüfdruck von 0,5 kPa) aufgebracht. Der Polymerfilm weist zusammen mit dem Klebstoff eine Wasserdampfdurchlässigkeit von ca. 1200 g/ m²/ 24 Std. auf (gemessen nach DIN EN 13726). Ein solcher Polymerfilm ist unter dem Handelsnamen Inspire 1305 bei der Fa. InteliCoat Technologies, Wrexham Industrial Estate, Wrexham LL13 9UF, UK, erhältlich. Die klebende Seite dieses Polymerfilms ist mit einem silikonisierten Abdeckpapier erhältlich unter dem Handelsnamen Separacon 980-60 bei der Fa. Maria Soell GmbH & Co. KG, Frankenstrasse 45, D-63667 Nidda-Eichelsdorf, abgedeckt. Auf der anderen, in Gebrauch körperabgewandten Seite des Polymerfilmes ist ein Applikationssystem angeordnet, das aus zwei Stützfolien besteht, auf denen jeweils eine Griffleiste angeordnet ist. Die Stützfolien sind wie in Figur 1 und Figur 2 dargestellt auf dem Polymerfilm angeordnet. In dem hier vorliegenden Folienverband ist jedoch zusätzlich ein Randbereich verwirklicht, der durch keine Stützfolie und keine Griffleiste abgedeckt ist. Dieser zusätzliche Randbereich ist auf der kurzen Seite des Rechtecks angeordnet und weist eine gleichförmige Breite von 5 mm auf. Die beiden Stützfolien sind gleich groß und weisen eine Kantenlänge von 57 x 36 mm auf (Auflagefläche: 2 x 20,5 cm² = 41,0 cm²). Der Abstand der beiden Folien beträgt in jedem Punkt ihrer zueinander parallel liegenden gleich langen Kanten ca. 3 mm. Damit ergibt sich insgesamt für beide Stützfolien eine Auflagefläche von 90 % bezogen auf Fläche der ersten Seite des Polymerfilms. Die Stützfolien sind aus einem 30 µm dicken Polyesterfilm gefertigt (gemessen bei einem Prüfdruck von 0,5 kPa). Auf jeder Stützfolie ist mittels eines Acrylat-Klebstoffes eine Griffleiste aufgeklebt. Die Griffleisten weisen im Querschnitt betrachtet eine Anordnung auf, wie sie mit Figur 4 wiedergegeben ist, wobei die erste mit Bezugszeichen (35) skizzierte Griffleiste eine Größe von 57 x 39 mm aufweist und über die gesamte Breite (57 mm) mit der zugehörigen Stützfolie verbunden ist. Die zweite mit Bezugszeichen (36) wiedergegeben Griffleiste weist eine Größe von 57 x 22 mm auf. Beide Griffleisten sind jeweils über einen 5 mm breiten adhäsiven Verbindungsstreifen mit der jeweiligen Stützfolie verbunden und sind aus einer 20 µm dicken transparenten Polyesterfolie gefertigt. Somit hat die erste Griffleiste eine Grifffläche, deren gleichförmige Breite 34 mm beträgt. Die Größe der Grifffläche der ersten Griffleiste beträgt 19,4 cm².

Die gleichförmige Breite der Grifffläche der zweiten Griffleiste beträgt 17 mm. Somit beträgt die Größe der Grifffläche der zweiten Griffleiste 9,7 cm². Die gleichförmige Breite des über die zweite Griffleiste überstehenden Teils der ersten Grifffläche, also die Breite der freien Grifffläche der ersten Griffleiste bemisst sich mit 9 mm. Die Größe der freien Grifffläche beträgt somit 5,1 cm².

Die Release-Eigenschaften der verwendeten Materialien dieses Ausführungsbeispiels 2 wurden an 60 x 80 mm großen Teststücken mittels der in DIN 53 530 beschriebenen Methode bestimmt. Die Prüfungen wurden bei einer Abzugsgeschwindigkeit von 300 mm/ min bestimmt. Demnach weist das Silikonpapier zum Polymerfilm eine Releasekraft von 0,77 N/ 25 mm auf wogegen der Stützfolie zum Polymerfilm eine Releasekraft von 0,09 N/ 25 mm aufweist. Damit sind die Release-Eigenschaften dieses Folienverbandes so eingestellt, dass die Abziehkraft, die notwendig ist, um eine Abdeckfolie von dem zu applizierenden Polymerfilm zu lösen, größer ist als die Abziehkraft, die notwendig ist, um die Stützfolie oder die Stützfolien von dem Polymerfilm zu trennen.

## Patentansprüche

1. Folienverband (20, 30, 50) umfassend einen Polymerfilm (1) und ein Applikationssystem zur leichteren Handhabung des Folienverbandes, wobei das Applikationssystem auf der ersten Seite des Polymerfilms (1) angeordnet ist und mindestens eine erste und eine zweite Stützfolie (22a, 22b, 32a, 32b, 32c, 52a, 52b) umfasst, **dadurch gekennzeichnet, dass** an die erste Stützfolie mindestens eine erste Griffleiste (25, 35, 55) und an die zweite Stützfolie mindestens eine zweite Griffleiste (26, 36, 56) angeformt ist, wobei die erste Griffleiste (25, 35, 55) eine Grifffläche zum Ergreifen der Griffleiste (25, 35, 55) aufweist und die erste Griffleiste (25, 35, 55) die zweite Griffleiste (26, 36, 56) zumindest abschnittsweise überfängt, wobei jede Griffleiste eine zusätzliche Materialkomponente ist und gleichzeitig mit mindestens einer Stützfolie von dem Polymerfilm entfernbar ist, und dass die durch die Stützfolien abgedeckte Fläche des Polymerfilms kleiner ist als die Fläche der ersten Seite des Polymerfilms, wobei die Auflagefläche der Stützfolien insgesamt weniger als 94 % der Fläche der ersten Seite des Polymerfilms entspricht.

2. Folienverband (20, 30, 50) nach Anspruch 1 **dadurch gekennzeichnet, dass** die erste Griffleiste (25, 35, 55) die zweite Griffleiste (26, 36, 56) vollständig überfängt und eine freie Grifffläche (251, 351, 551) von mindestens 2 cm² aufweist.

3. Folienverband (20, 30, 50) nach mindestens einem der vorgenannten Ansprüche **dadurch gekennzeichnet, dass** der Polymerfilm (1) mindestens einen stützfolienfreien Bereich (27, 37a, 37b, 38a, 38b, 57, 58) aufweist.

4. Folienverband (20, 30, 50) nach mindestens einem der vorgenannten Ansprüche **dadurch gekennzeichnet, dass** die erste oder die zweite Griffleiste (25, 26, 35, 36, 55, 56) einen ersten stützfolienfreien Bereich (27, 37a, 37b, 57) zumindest abschnittsweise überfängt.

5. Folienverband (20, 30, 50) nach mindestens einem der vorgenannten Ansprüche **dadurch gekennzeichnet, dass** das Applikationssystem dieselbe Größe aufweist, wie der Polymerfilm (1).

6. Folienverband (20, 30, 50) nach mindestens einem der vorgenannten Ansprüche **dadurch gekennzeichnet, dass** der Polymerfilm (1) einen stützfolienfreien Randbereich (17, 38a, 38b, 58) aufweist.

7. Folienverband (20, 30, 50) nach mindestens einem der vorgenannten Ansprüche **dadurch gekennzeichnet, dass** ein erster stützfolienfreier Bereich (27, 37a, 37b, 57) des Polymerfilms (1) zwischen der ersten und der zweiten Stützfolie liegt.

8. Folienverband (20, 30, 50) nach mindestens einem der vorgenannten Ansprüche **dadurch gekennzeichnet, dass** der Polymerfilm (1) mindestens zwei stützfolienfreie Bereiche (37a, 37b 38a, 38b, 57, 58) aufweist, die voneinander getrennt sind.

9. Folienverband (20, 30, 50) nach mindestens einem der vorgenannten Ansprüche **dadurch gekennzeichnet, dass** die Stützfolien (22a, 22b, 32a, 32b, 32c, 52a, 52b) und die Griffleisten (25, 26, 35, 36, 55, 56) aus gleichen Materialien bestehen.

10. Folienverband (20, 30, 50) nach mindestens einem der vorgenannten Ansprüche **dadurch gekennzeichnet, dass** die erste Griffleiste (25, 35, 55) eine höhere Steifigkeit aufweist als die zweite Griffleiste (26, 36; 56).

11. Folienverband (20, 30, 50) nach mindestens einem der vorgenannten Ansprüche **dadurch gekennzeichnet, dass** die zweite Seite des Polymerfilmes (1) mit einem Haftklebemittel (2), insbesondere vollflächig mit einem Haftklebemittel (2) beschichtet ist und das Haftklebemittel (2) mit einer Releasefolie oder -papier (3) abgedeckt ist.

12. Folienverband (20, 30, 50) nach mindestens einem der vorgenannten Ansprüche **dadurch gekennzeichnet, dass** auf der zweiten Seite des Polymerfilms (1) ein Wundkissen aufgebracht ist.

13. Folienverband (20, 30, 50) nach mindestens einem der vorgenannten Ansprüche **dadurch gekennzeichnet, dass** auf der zweiten Seite des Polymerfilms (1) eine die Wundheilung fördernde Schicht aufgebracht ist.

14. Folienverband (20, 30, 50) nach mindestens einem der vorgenannten Ansprüche **dadurch gekennzeichnet, dass** der Polymerfilm (1) ein Polyurethanfilm, Polyesterurethanfilm oder Polyetherurethanfilm ist.

15. Folienverband (20, 30, 50) nach mindestens einem der vorgenannten Ansprüche **dadurch gekennzeichnet, dass** die Stützfolien in einer Ebene angeordnet sind.

## Claims

1. A film dressing (20, 30, 50) comprising a polymer film (1) and an application system for permitting an improved ease of use of said film dressing, in which the application system is located on a first side of the polymer film (1) and comprises at least one first and one second supporting film (22a, 22b, 32a, 32b, 32c, 52a, 52b), **characterized in that** at least one first grip strip (25, 35, 55) is formed on the first supporting film and at least one second grip strip (26, 36, 56) is formed on the second supporting film, in which at least the first grip strip (25, 35, 55) comprises a grip surface that enables the grip strip (25, 35, 55) to be grasped and the first grip strip (25, 35, 55) at least partially overlaps the second grip strip (26, 36, 56), each grip strip being an additional material component and being removable from the polymer film simultaneously with at least one supporting film, and **in that** the area of the polymer film which is covered by the supporting films is smaller than the area of the first side of the polymer film, where the combined contact area of the supporting films is less than 94% of the area of the first side of the polymer film.

2. Film dressing (20, 30, 50) according to Claim 1, **characterized in that** the first grip strip (25, 35, 55) completely overlaps the second grip strip (26, 36, 56) and comprises an exposed grip surface (251, 351, 551) of at least 2 cm².

3. Film dressing (20, 30, 50) according to at least one of the previous claims, **characterized in that** the polymer film (1) comprises at least an area without supporting films (27, 37a, 37b, 38a, 38b, 57, 58).

4. Film dressing (20, 30, 50) according to at least one of the previous claims, **characterized in that** the first or the second grip strip (25, 26, 35, 36, 55, 56) overlaps at least partially a first area without supporting films (27, 37a, 37b, 57).

5. Film dressing (20, 30, 50) according to at least one of the previous claims, **characterized in that** the application system comprises the same size as the polymer film (1).

6. Film dressing (20, 30, 50) according to at least one of the previous claims, **characterized in that** the polymer film (1) comprises a peripheral area without supporting films (17, 38a, 38b, 58).

7. Film dressing (20, 30, 50) according to at least one of the previous claims, **characterized in that** a first area without supporting films (27, 37a, 37b, 57) of the polymer film (1) is positioned between the first and the second supporting film.

8. Film dressing (20, 30, 50) according to at least one of the previous claims, **characterized in that** the polymer film (1) comprises at least two areas without supporting films (37a, 37b, 38a, 38b, 57, 58) that are separated from one another.

9. Film dressing (20, 30, 50) according to at least one of the previous claims, **characterized in that** the supporting films (22a, 22b, 32a, 32b, 32c, 52a, 52b) and the grip strips (25, 26, 35, 36, 55, 56) are comprised of identical materials.

10. Film dressing (20, 30, 50) according to at least one of the previous claims, **characterized in that** the first grip strip (25, 35, 55) is more rigid than the second grip strip (26, 36, 56).

11. Film dressing (20, 30, 50) according to at least one of the previous claims, **characterized in that** the second side of the polymer film (1) is coated with an adhesive material (2), particularly continuously with an adhesive material (2) and the adhesive material (2) is covered with a release film or paper (3).

12. Film dressing (20, 30, 50) according to at least one of the previous claims, **characterized in that** a wound pad is attached to the second side of the polymer film (1).

13. Film dressing (20, 30, 50) according to at least one of the previous claims, **characterized in that** a layer that promotes the healing of the wound is attached to the second side of the polymer film (1) .

14. Film dressing (20, 30, 50) according to at least one of the previous claims, **characterized in that** the polymer film (1) is comprised of a polyurethane film, polyester urethane film or polyether urethane film.

15. Film dressing (20, 30, 50) according to at least one of the previous claims, **characterized in that** the supporting films are positioned on one level.

## Revendications

1. Assemblage de films (20, 30, 50) comprenant un film polymère (1) et un système d'application pour une manipulation plus aisée de l'assemblage de films, dans lequel le système d'application est disposé sur la première face du film polymère (1) et comprend au moins un premier et un deuxième films de support (22a, 22b, 32a, 32b, 32c, 52a, 52b), **caractérisé en ce qu'**au moins une première lèvre de saisie (25, 35, 55) est formée sur le premier film de support et au moins une deuxième lèvre de saisie (26, 36, 56) est formée sur le deuxième film de support, dans lequel la première lèvre de saisie (25, 35, 55) présente une première surface de saisie pour saisir la lèvre de saisie (25, 35, 55) et la première lèvre de saisie (25, 35, 55) recouvre au moins localement la deuxième lèvre de saisie (26, 36, 56), dans lequel chaque lèvre de saisie est un composant de matière additionnel et peut être enlevée du film polymère en même temps qu'au moins un film de support, et **en ce que** la surface du film polymère masquée par les films de support est plus petite que la surface de la première face du film polymère, la surface d'appui des films de support correspondant au total à moins de 94 % de la surface de la première face du film polymère.

2. Assemblage de films (20, 30, 50) selon la revendication 1, **caractérisé en ce que** la première lèvre de saisie (25, 35, 55) recouvre entièrement la deuxième lèvre de saisie (26, 36, 56) et présente une surface de saisie libre (251, 351, 551) d'au moins 2 cm² .

3. Assemblage de films (20, 30, 50) selon au moins une des revendications précédentes, **caractérisé en ce que** le film polymère (1) présente au moins une zone exempte de film de support (27, 37a, 37b, 38a, 38b, 57, 58).

4. Assemblage de films (20, 30, 50) selon au moins une des revendications précédentes, **caractérisé en ce que** la première ou la deuxième lèvre de saisie (25, 26, 35, 36, 55, 56) recouvre au moins localement une première zone exempte de film de support (27, 37a, 37b, 57).

5. Assemblage de films (20, 30, 50) selon au moins une des revendications précédentes, **caractérisé en ce que** le système d'application présente la même grandeur que le film polymère (1).

6. Assemblage de films (20, 30, 50) selon au moins une des revendications précédentes, **caractérisé en ce que** le film polymère (1) présente une zone de bord exempte de film de support (17, 38a, 38b, 58).

7. Assemblage de films (20, 30, 50) selon au moins une des revendications précédentes, **caractérisé en ce qu'**une première zone exempte de film de support (27, 37a, 37b, 57) du film polymère (1) est située entre le premier et le deuxième films de support.

8. Assemblage de films (20, 30, 50) selon au moins une des revendications précédentes, **caractérisé en ce que** le film polymère (1) présente au moins deux zones exemptes de film de support (37a, 37b, 38a, 38b, 57, 58), qui sont séparées l'une de l'autre.

9. Assemblage de films (20, 30, 50) selon au moins une des revendications précédentes, **caractérisé en ce que** les films de support (22a, 22b, 32a, 32b, 32c, 52a, 52b) et les lèvres de saisie (25, 26, 35, 36, 55, 56) sont constitués des mêmes matières.

10. Assemblage de films (20, 30, 50) selon au moins une des revendications précédentes, **caractérisé en ce que** la première lèvre de saisie (25, 35, 55) présente une plus grande rigidité que la deuxième lèvre de saisie (26, 36, 56).

11. Assemblage de films (20, 30, 50) selon au moins une des revendications précédentes, **caractérisé en ce que** la deuxième face du film polymère (1) est revêtue d'un agent autoadhésif (2), en particulier d'un agent autoadhésif (2) sur toute la surface, et l'agent autoadhésif (2) est masqué par un film ou un papier antiadhésif (3).

12. Assemblage de films (20, 30, 50) selon au moins une des revendications précédentes, **caractérisé en ce qu'**un tampon cicatrisant est appliqué que la deuxième face du film polymère (1).

13. Assemblage de films (20, 30, 50) selon au moins une des revendications précédentes, **caractérisé en ce qu'**une couche favorisant la cicatrisation est appliquée sur la deuxième face du film polymère (1).

14. Assemblage de films (20, 30, 50) selon au moins une des revendications précédentes, **caractérisé en ce que** le film polymère (1) est un film de polyuréthane, un film de polyesteruréthane ou un film de polyétheruréthane.

15. Assemblage de films (20, 30, 50) selon au moins une des revendications précédentes, **caractérisé en ce que** les films de support sont disposés dans un plan.
